# EUROPEAN PATENT APPLICATION

(11) **EP 2 158 876 A1**
(43) Date of publication of application: **03.03.2010**
(21) Application number: 09011276.4
(22) Date of filing: 02.09.2009
(51) Int. Cl.: A61F 2/30

(54) **Plug for covering screw holes in prostethic implants**

(30) Priority: 02.09.2008 US 93468 P
(71) Applicant: Zimmer GmbH, 8404 Winterthur (CH)
(72) Inventor: Breimesser, Hermann, 8353 Elgg (CH); Devanthey, Cedric, 8910 Affoltern am Albis (CH)
(74) Representative: Manitz, Finsterwald & Partner GbR

(57) **Abstract**

A plug (24) for covering an aperture (20) in a prosthetic implant and a method for using the same. The aperture may include a screw hole designed to receive a bone screw for securing the implant to a patient's bone. When the aperture does not receive a bone screw, the plug is configured to fit within the aperture to prevent materials, such as bone debris and adhesive, from seeping through the aperture and onto a receiving surface of the implant.

## Description

### CROSS REFERENCE TO RELATED APPLICATION

This application claims priority from Provisional Patent Application No. 61/093,468, entitled "Plug for Covering Screw Holes in Prosthetic Implants," filed on September 2, 2008 by the same inventors hereof, the disclosure of which is expressly incorporated herein by reference.

### BACKGROUND

### 1. Field of the Invention.

The present invention relates to prosthetic implants. More particularly, the present invention relates to a plug for covering screw holes in prosthetic implants and to a method for using the same.

### 2. Description of the Related Art.

Joint arthroplasty is a surgical procedure for replacing damaged components of a joint with prosthetic components. Such damage may be caused by, for example, traumatic injury or some form of arthritis, such as osteoarthritis. Joint arthroplasty may relieve pain and restore motion in the damaged joint.

The hip joint is formed between the head of the femur and the acetabulum of the pelvis. Arthroplasty of the hip joint may involve replacing the acetabulum with a prosthetic implant. A prosthetic acetabulum can include a convex acetabular shell that engages a patient's pelvis and a concave liner positioned within the acetabular shell that receives the head of the femur itself or a prosthetic femoral head.

Various methods are available for securing the prosthetic acetabulum to the patient's pelvis, such as driving screws into the bone and applying adhesive. Acetabular shells designed to be secured with bone screws include numerous screw holes through which the screws can be driven into the patient's bone. Depending on the condition of the patient's pelvic bone and/or the surgeon's preference, some screw holes may receive a bone screw while others may not. If left open, materials such as bone debris and adhesive may seep through the screw holes.

Plugs that are currently available for covering the screw holes of a prosthetic acetabulum present several disadvantages. For example, current plugs cannot be removed after insertion into the screw hole. If a surgeon plugs a screw hole and later chooses to insert a screw through the hole, the entire acetabular shell would have to be removed and replaced. Also, current plugs have bending parts that may become deformed or break, which in turn affects the plug's ability to function properly.

### SUMMARY

The present invention relates to a plug for covering an aperture in a prosthetic implant and a method for using the same. The aperture may include a screw hole designed to receive a bone screw for securing the implant to a patient's bone. When the aperture does not receive a bone screw, the plug is configured to fit within the aperture to prevent materials, such as bone debris and adhesive, from seeping through the aperture and onto a receiving surface of the implant.

According to an embodiment of the present invention, a plug is provided for covering an aperture in a prosthetic implant. The prosthetic implant includes an annular ridge projecting into the aperture and narrowing the aperture. The plug includes a distal end, a proximal end, a head located at the proximal end of the plug, and a body extending between the head of the plug and the distal end of the plug. The body includes an exterior surface and a longitudinal axis. The plug further includes a thread that extends from the exterior surface of the body and wraps helically around the body and at least one notch in the distal end of the plug that interrupts the thread. The plug has a first position within the aperture in which the annular ridge aligns with the plug along the notch and a second position within the aperture in which the thread engages the annular ridge.

According to another embodiment of the present invention, an implant system is provided. The implant system includes a prosthetic implant and a plug. The prosthetic implant includes a bone-contacting surface, a receiving surface opposite the bone-contacting surface, and at least one aperture extending a length between the bone-contacting surface and the receiving surface. The plug is sized to be received within the at least one aperture of the prosthetic implant. The plug includes a distal end, a proximal end, and a body extending between the proximal end of the plug and the distal end of the plug a length substantially equivalent to the length of the aperture. The body includes an exterior surface and a longitudinal axis. The plug further includes a thread that wraps helically around the body and at least one notch in the body that interrupts the thread.

According to yet another embodiment of the present invention, a method is provided for covering an aperture in a prosthetic implant. The prosthetic implant includes an annular ridge projecting into the aperture and narrowing the aperture. The method includes the steps of providing a plug having a proximal end, a distal end, a threaded portion, and an interrupted thread portion, positioning the plug within the aperture of the prosthetic implant to align the interrupted thread portion of the plug with the annular ridge, and turning the plug to engage the annular ridge with the threaded portion of the plug.

### BRIEF DESCRIPTION OF THE DRAWINGS

The above-mentioned and other features and advantages of this invention, and the manner of attaining them, will become more apparent and the invention itself will be better understood by reference to the following description of embodiments of the invention taken in conjunction with the accompanying drawings, wherein:
Figure 1 is a cross-sectional view of a prosthetic acetabulum and a plug of the present invention;
Figure 2 is a partial cross-sectional view of the prosthetic acetabulum of Figure 1 showing an aperture;
Figure 3 is a view similar to Figure 2 showing a bone screw in the aperture;
Figure 4 is a view similar to Figure 2 showing a plug of the present invention in the aperture;
Figure 5 is a distal plan view of a plug of the present invention;
Figure 6 is a proximal plan view of the plug of Figure 5;
Figure 7 is a cross-sectional view of the plug of Figure 6, taken along line 7-7 of Figure 6;
Figure 7A is a partial cross-sectional view of the plug of Figure 7;
Figure 8 is a distal plan view of another plug of the present invention;
Figure 9 is a distal plan view of yet another plug of the present invention;
Figure 10 is a proximal plan view of the plug of Figure 9;
Figure 11 is a cross-sectional view of the plug of Figure 10, taken along line 11-11 of Figure 10;
Figure 11A is a partial cross-sectional view of the plug of Figure 11;
Figure 11B is a partial distal plan view of the plug of Figure 11;
Figure 12 is a distal plan view of still yet another plug of the present invention;
Figure 13 is a proximal plan view of the plug of Figure 12;
Figure 14 is a cross-sectional view of the plug of Figure 13, taken along line 14-14 of Figure 13;
Figure 14A is a partial cross-sectional view of the plug of Figure 14; and
Figure 14B is a partial distal plan view of the plug of Figure 14.

Corresponding reference characters indicate corresponding parts throughout the several views. The exemplifications set out herein illustrate exemplary embodiments of the invention and such exemplifications are not to be construed as limiting the scope of the invention in any manner.

### DETAILED DESCRIPTION

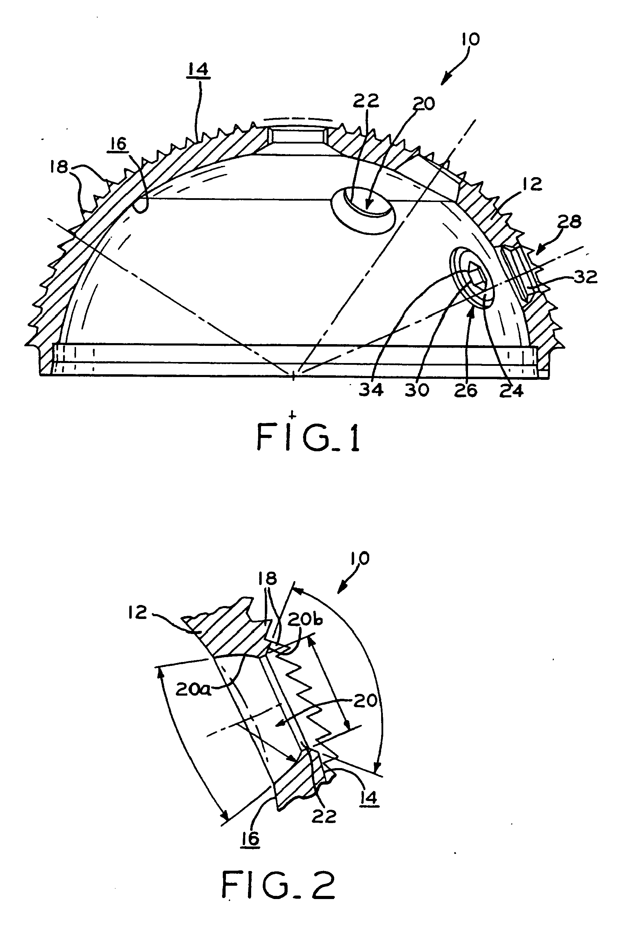

Referring to Figure 1, an exemplary medical implant is shown in the form of prosthetic acetabulum 10 of the type that is implanted within the acetabulum of a patient's pelvis during a partial or total hip arthroplasty procedure. Prosthetic acetabulum 10 generally provides a concave bearing surface that receives either the convex head of a femur or a convex prosthetic femoral head. Although the present invention is described herein in the form of prosthetic acetabulum 10, the present invention is generally applicable to any type of medical implant having apertures that may be sealed after implantation of the implant system into the body.

Referring to Figures 1-4, prosthetic acetabulum 10 may include acetabular shell 12 and a liner (not shown). Acetabular shell 12 includes an exterior bone-contacting surface 14 and an interior receiving surface 16. When prosthetic acetabulum 10 includes a liner, receiving surface 16 of acetabular shell 12 is sized to receive and attach to the liner. The liner has a concave bearing surface that receives either the head of the femur or the prosthetic femoral head. If a separate liner is not utilized, receiving surface 16 of acetabular shell 12 itself may be designed as a bearing surface capable of receiving the head of the femur or a prosthetic femoral head.

Referring still to Figures 1-4, bone-contacting surface 14 of acetabular shell 12 is located opposite receiving surface 16. Upon implantation into a patient, bone-contacting surface 14 is positioned against the patient's pelvic bone. Various methods are available for securing acetabular shell 12 to the patient's pelvic bone, such as driving screws into the bone and applying adhesive, for example. Bone-contacting surface 14 may further include a textured finish and/or teeth 18 to enhance the attachment between acetabular shell 12 and the patient's bone. The distance between bone-contacting surface 14 and receiving surface 16, or the thickness or acetabular shell 12, may be as small as approximately 1, 2, or 3 millimeters or as large as approximately 4, 5, or 6 millimeters.

Referring to Figures 2-4, acetabular shell 12 includes at least one aperture 20 that extends through acetabular shell 12 from bone-contacting surface 14 to receiving surface 16. The length of aperture 20 depends on the thickness of acetabular shell 12, or the distance between bone-contacting surface 14 and receiving surface 16 of acetabular shell 12. Acetabular shell 12 is designed to receive bone screw 100 within aperture 20 for attaching acetabular shell 12 to the patient's bone. Aperture 20 may be defined between proximal wall 20a and distal wall 20b, both of which are described in more detail below. Proximal wall 20a and distal wall 20b surrounding aperture 20 are unthreaded to permit bone screw 100 to be oriented in various positions within aperture 20.

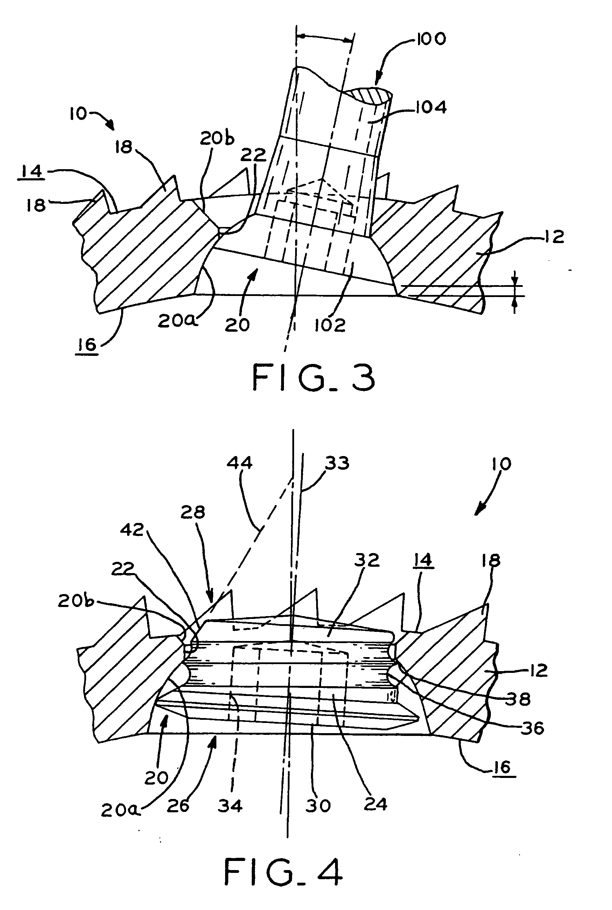

Proximal wall 20a of aperture 20 is located near receiving surface 16 of acetabular shell 12. As shown in Figure 3, proximal wall 20a surrounds aperture 20 to provide a location for head 102 of bone screw 100 within aperture 20. Proximal wall 20a defines aperture 20 that is essentially frusto-conical in shape, with aperture 20 narrowing as it extends away from receiving surface 16 and toward bone-contacting surface 14 of acetabular shell 12. The size and shape of proximal wall 20a permits head 102 of bone screw 100 to be oriented in various positions within aperture 20, as shown in Figure 3. The narrowing of aperture 20, in particular, prevents head 102 of bone screw 100 from pulling out of aperture 20. Therefore, the narrowing of aperture 20 prevents acetabular shell 12 from separating from the patient's bone when secured with bone screw 100.

Distal wall 20b of aperture 20 is located near bone-contacting surface 14 of acetabular shell 12 and adjacent to proximal wall 20a of aperture 20. Distal wall 20b defines aperture 20 that is essentially frusto-conical in shape, with aperture 20 widening as it extends toward bone-contacting surface 14 and away from receiving surface 16 of acetabular shell 12. The widening of aperture 20 permits shaft 104 of bone screw 100 to extend away from acetabular shell 12 at various angles. Plugs can be designed to attach to acetabular shell 12 by expanding outwardly toward distal wall 20b of aperture 20.

Between proximal wall 20a and distal wall 20b, acetabular shell 12 includes annular ridge 22. Ridge 22 projects into aperture 20 to define a narrowed portion of aperture 20. As discussed above with reference to Figure 3, ridge 22 is provided to prevent head 102 of bone screw 100 from pulling out of aperture 20. Ridge 22 may have a thickness T as small as approximately 0.3, 0.4, or 0.5 millimeters or as large as approximately 0.6, 0.7, or 0.8 millimeters. Ridge 22 may define a narrowed portion of aperture 20 having a diameter as small as approximately 4, 5, or 6 millimeters or as large as approximately 7, 8, or 9 millimeters

Referring to Figure 4, plug 24 is provided to cover aperture 20 of acetabular shell 12 when aperture 20 does not receive bone screw 100 (Figure 3). Plug 24 is configured to fit within aperture 20 to prevent materials, such as bone debris and adhesive used to secure bone-contacting surface 14 of acetabular shell 12 to the patient's bone, from seeping through aperture 20 and onto receiving surface 16 of acetabular shell 12.

Plug 24 may be constructed of metal, such as titanium or a titanium alloy, polymer, or another biocompatible material. In an exemplary embodiment of the present invention, plug 24 is constructed of commercially pure titanium (cpTi), such as Protasul-Ti. Plug 24 may be manufactured by any method known in the art, such as molding.

Referring still to Figure 4, plug 24 includes proximal end 26, distal end 28, and longitudinal axis 33 extending from proximal end 26 to distal end 28. As used herein, "proximal" and "distal" are determined relative to a surgeon or another user, such that distal end 28 of plug 24 is farther from the surgeon than proximal end 26 of plug 24 during use. Plug 24 is slightly frusto-conical in shape to mimic the shape of aperture 20, and in particular aperture 20 as defined within proximal wall 20a. Near its proximal end 26, plug 24 may have a diameter as small as approximately 6, 7, or 8 millimeters or as large as approximately 9, 10, or 11 millimeters. Plug 24 may narrow toward distal end 28. Near its distal end 28, plug 24 may have a diameter as small as approximately 4, 5, or 6 millimeters or as large as approximately 7, 8, or 9 millimeters.

Plug 24 further includes head 30 located near proximal end 26. Head 30 includes bore 34 configured to cooperate with a tool (not shown) for turning plug 24. In an exemplary embodiment of the present invention, bore 34 is hexagonal to receive a hex wrench, also known as an Allen wrench.

Plug 24 further includes body 32 extending from head 30 to distal end 28. Body 32 of plug 24 includes exterior surface 36 that extends the length of body 32. Body 32 also includes thread 38 that projects from exterior surface 36 and wraps helically around body 32. Thread 38 is sized to engage ridge 22 of acetabular shell 12 when plug 24 is positioned within aperture 20. Therefore, adjacent threads are separated by a distance at least slightly exceeding the thickness of ridge 22.

Referring to Figures 3-4, unlike bone screw 100, which is driven into adjacent bone to secure acetabular shell 12 in place, plug 24 is not driven into the patient's bone. In an exemplary embodiment of the present invention, illustrated in Figure 4, plug 24 is sized to avoid direct contact with the patient's bone. Distal end 28 of plug 24 may be relatively flat or may protrude slightly, as shown in Figure 4, to avoid protruding beyond bone-contacting surface 14 of acetabular shell 12, and in particular beyond teeth 18 on bone-contacting surface 14 of acetabular shell 12. Similarly, plug is sized to avoid direct contact with an acetabular liner (not shown) or a femoral head (not shown) received within acetabular shell 12. Proximal end 26 of plug 24 may be relatively flat to avoid protruding beyond receiving surface 16 of acetabular shell 12. As mentioned above, the distance between bone-contacting surface 14 and receiving surface 16 of acetabular shell 12 may be as small as approximately 1, 2, or 3 millimeters or as large as approximately 4, 5, or 6 millimeters. In order for plug 24 to avoid protruding from aperture 20 beyond bone-contacting surface 14 and/or receiving surface 16, plug 24 may be provided in a size corresponding to the thickness of acetabular shell 12. Therefore, the distance between proximal end 26 and distal end 28 of plug 24 may be as small as approximately 1, 2, or 3 millimeters or as large as approximately 4, 5, or 6 millimeters.

Referring to Figure 4, thread 38 is interrupted near distal end 28 of plug 24 by at least one notch 42 in distal end 28 of body 32. In an exemplary embodiment, approximately one or two layers of thread 38 are interrupted by notch 42 in distal end 28 of body 32. Notch 42 is essentially an indentation in or cut-away from distal end 28 of body 32. Notch 42 may be located at least partially within transverse plane 44. Transverse plane 44 extends transversely to longitudinal axis 33, intersecting both exterior surface 36 of body 32 and distal end 28 of body 32.

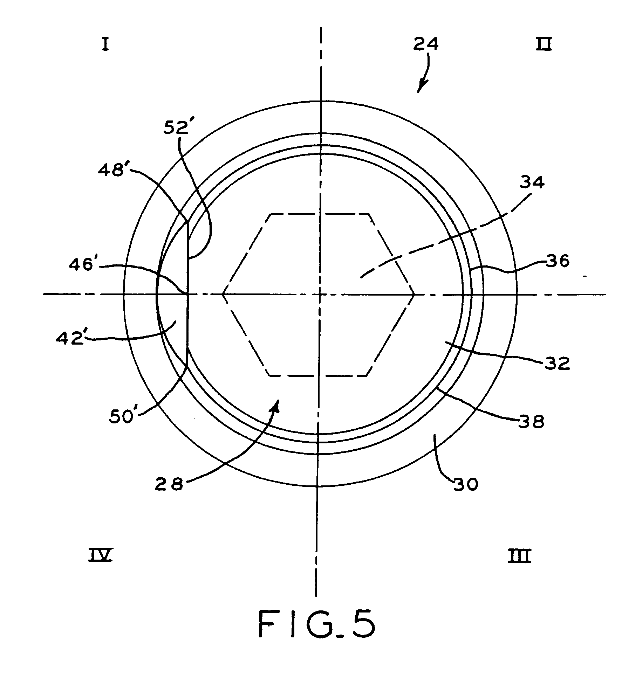

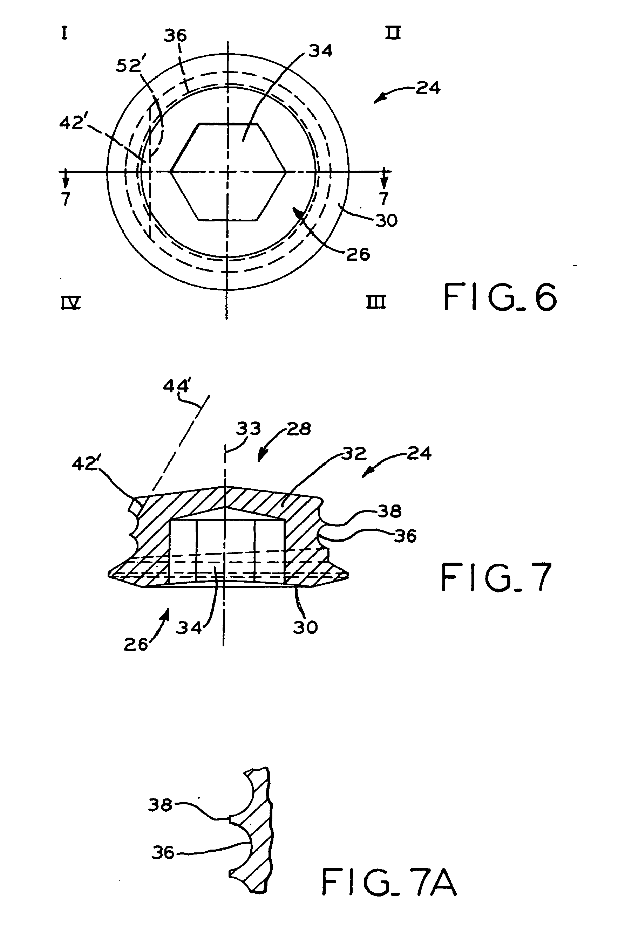

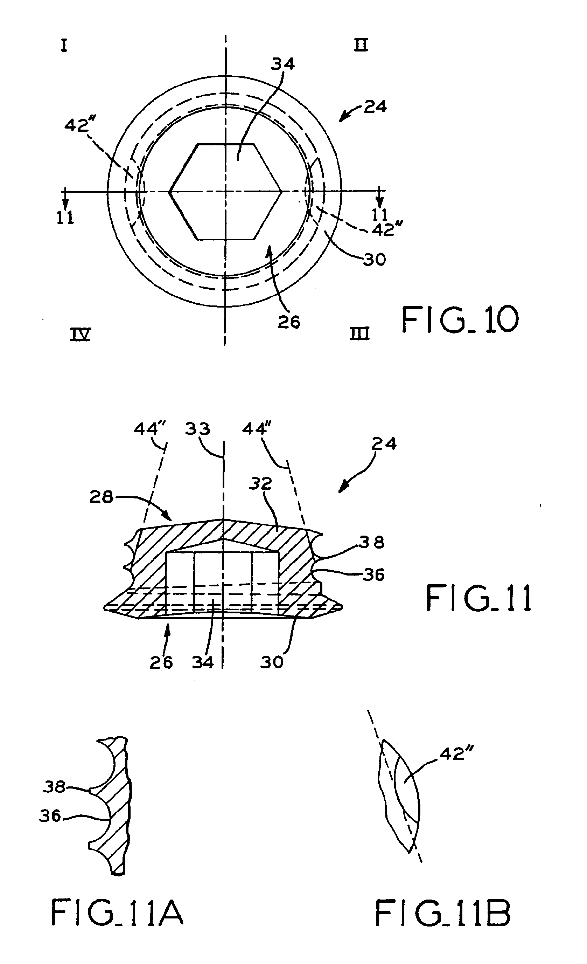

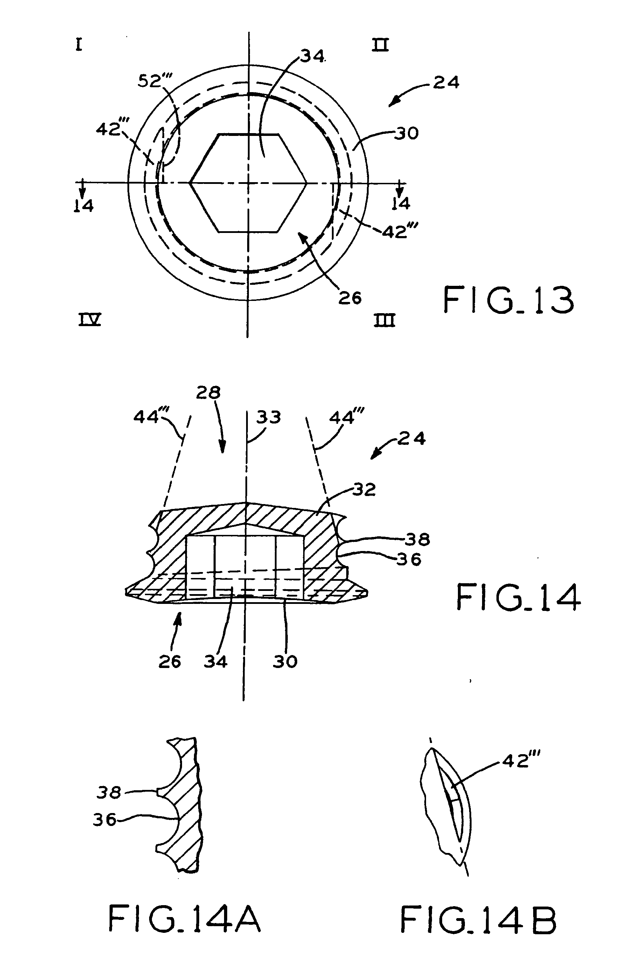

For purposes of describing notch 42 in more detail, plug 24 is shown divided into quadrants I, II, III, and IV in Figures 5-14. These quadrants are illustrative only, as the position of the notch is not limited to the locations set forth below.

In the following embodiments of plug 24, each notch 42'-42"' includes a respective central portion 46'-46"', end portion 48'-48", and end portion 50'-50"'. End portions 48'-48" and end portions 50'-50"' are located on either side of the respective central portions 46'-46"'. The intersections between transverse planes 44'-44"' and distal end 28 of each body 32 forms distal intersections 52'-52"'.

According to an embodiment of the present invention, illustrated in Figures 4-8, notch 42' is located entirely within transverse plane 44', and notch 42' extends the length of distal intersection 52'. In this embodiment, transverse plane 44' may extend approximately 30 degrees from longitudinal axis 33 within quadrants I and IV. More specifically, transverse plane 44' extends from exterior surface 36 of body 32 within quadrants I and IV to distal end 28 of body 32 within quadrants I and IV. Because notch 42' is located entirely within transverse plane 44', central portion 46' and end portions, 48' and 50', of notch 42' are all located within transverse plane 44'. Further, notch 42' extends the length of distal intersection 52' between distal end 28 of body 32 and transverse plane 44'. In this embodiment, transverse plane 44' is tangential to body 32 along notch 42'. As illustrated, notch 42' is located within transverse plane 44', which is located within quadrants I and IV. However, notch 42' and transverse plane 44' could be located within a single quadrant or could span several quadrants.

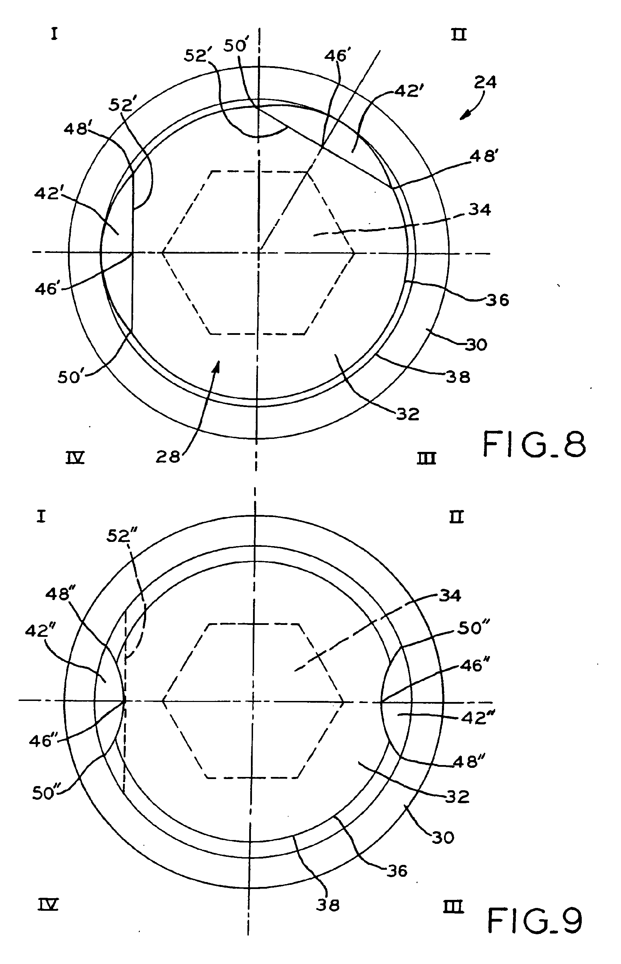

According to another embodiment of the present invention, illustrated in Figures 9-11, notch 42" is located only partially within transverse plane 44". In this embodiment, transverse plane 44" may extend approximately 15 degrees from longitudinal axis 33 within quadrants I and IV. (A second transverse plane 44" is located within quadrants II and III.) More specifically, transverse plane 44" within quadrants I and IV extends from exterior surface 36 of body 32 within quadrants I and IV to distal end 28 of body 32 within quadrants I and IV. (Similarly, transverse plane 44" within quadrants II and III extends from exterior surface 36 of body 32 within quadrants II and III to distal end 28 of body 32 within quadrants II and III.) Unlike notch 42' which is located entirely within transverse plane 44' (Figure 4), notch 42" curves toward exterior surface 36 of body 32. In an exemplary embodiment, central portion 46" of notch 42" is located within transverse plane 44", while end portions, 48" and 50", of notch 42" curve away from longitudinal axis 33 and out of transverse plane 44". As illustrated, notch 42" is located partially within transverse plane 44", which is located within quadrants I and IV. (A second notch 42" is located within a second transverse plane 44", which is located within quadrants II and III.) However, each notch 42" and transverse plane 44" could be located within a single quadrant or could span several quadrants.

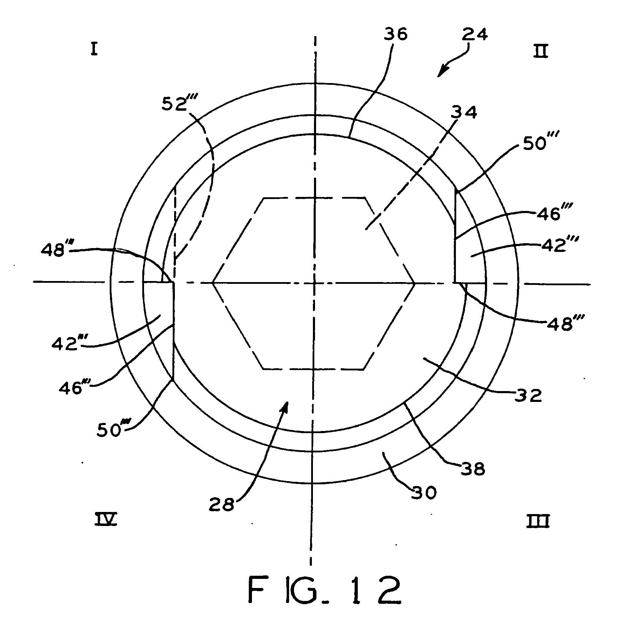

According to yet another embodiment of the present invention, illustrated in Figures 12- 14, notch 42"' is located entirely within transverse plane 44''' and extends only partially the length of distal intersection 52"'. In this embodiment, transverse plane 44"' may extend approximately 15 degrees from longitudinal axis 33 within quadrants I and IV. (A second transverse plane 44"' is located within quadrants II and III.) More specifically, transverse plane 44"' within quadrants I and IV extends from exterior surface 36 of body 32 within quadrants I and IV to distal end 28 of body 32 within quadrants I and IV. (Similarly, transverse plane 44''' within quadrants II and III extends from exterior surface 36 of body 32 within quadrants II and III to distal end 28 of body 32 within quadrants II and III.) Like notch 42' (Figure 4), all of notch 42"' is located within transverse plane 44"'. However, unlike notch 42' (Figure 4), notch 42"' does not extend the entire length of distal intersection 52"' between distal end 28 of body 32 and transverse plane 44"'. More specifically, central portion 46"' and end portions, 48''' and 50"', are all located within transverse plane 44''', but end portions, 48''' and 50"', are not located at the edges of distal intersection 52''' within quadrants I and IV. (Similarly, end portions, 48''' and 50"', are not located at the edges of distal intersection 52"' within quadrants II and III.)
As illustrated, although transverse plane 44''' spans quadrants I and IV, notch 42" is located only within quadrant IV. (A second transverse plane 44''' spans quadrants II and III, but a second notch 42"' is located only within quadrant II.) Each notch 42"' could, however, extend into multiple quadrants.

Referring to Figures 8, 9, and 12, plug 24 may include multiple notches. The notches may be spaced apart from one another in various arrangements. For example, the notches may be aligned symmetrically on distal end 28 of plug 24. As shown in Figure 9, notches 42" are located 180 degrees apart from one another on distal end 28 of plug 24. However, the notches need not be aligned symmetrically. As shown in Figure 8, notches 42' are located approximately 120 degrees apart from one another on distal end 28 of plug 24.

Referring to Figure 3, a method is provided for covering aperture 20 of acetabular shell 12 with plug 24 when aperture 20 does not receive bone screw 100. Acetabular shell 12 may be secured in place by driving bone screw 100 through apertures 20 in acetabular shell 12 and/or by applying adhesive between acetabular shell 12 and the patient's pelvic bone, for example. Depending on the surgeon's preference and/or the condition of the patient's pelvic bone, not all apertures 20 may receive bone screws.

Referring to Figure 4, the present method involves positioning plug 24 within aperture 20 such that the unthreaded portion of plug 24 is aligned with ridge 22. More specifically, this step involves positioning plug 24 within aperture 20, such as via press-fitting plug 24 into aperture 20, with distal end 28 of plug 24 facing bone-contacting surface 14 of acetabular shell 12 and with proximal end 26 of plug 24 facing receiving surface 16 of acetabular shell 12. In this position, head 30 of plug 24 is accessible from receiving surface 16 of acetabular shell 12, while distal end 28 of plug 24 extends into aperture 20 and approaches ridge 22. As distal end 28 of plug 24 approaches ridge 22 of acetabular shell 12, ridge 22 of acetabular shell 12 aligns with plug 24 along notch 42. Pressure may be applied to proximal end 26 of plug 24 until plug 24 engages ridge 22 along notch 42. It is within the scope of the present invention that acetabular shell 12 may be provided with plug 24 already in this position.

Referring still to Figure 4, the present method further involves turning plug 24 so that thread 38 engages ridge 22. Plug 24 need not be turned a full turn, or 360 degrees, to engage ridge 22. This step involves using a tool, such as a hex wrench, that cooperates with bore 34 in head 30 of plug 24. In an exemplary embodiment of the present invention, when plug 24 reaches its final position within aperture 20, plug 24 essentially fills aperture 20 to prevent materials, such as bone debris and adhesive, from seeping through aperture 20 and onto receiving surface 16 of acetabular shell 12. Also in this exemplary embodiment, plug 24 is sized to fit almost entirely within aperture 20, such that head 30 on proximal end 26 of plug 24 does not project beyond receiving surface 16 of acetabular shell 12 and such that distal end 28 of plug 24 does not project beyond bone-contacting surface 14 of acetabular shell 12.

[0001] Advantageously, the present invention provides a robust design for plug 24 that does not require bendable or movable parts to engage ridge 22 of acetabular shell 12. Also, plug 24 may be removed once inserted into aperture 20, similar to a typical screw. In addition, plug 24 is capable of being secured within an unthreaded aperture 20 by engaging ridge 22. Without notch 42 in plug 24, a surgeon may struggle to engage ridge 22 at all. Finally, plug 24 is designed to engage ridge 22 while still being short enough in length to avoid projecting well beyond bone-contacting surface 14 of acetabular shell 12 and receiving surface 16 of acetabular shell 12.

According to an embodiment of the invention, the body of the plug may comprise titanium.

A method of covering an aperture in a prosthetic implant, the prosthetic implant including an annular ridge projecting into the aperture and narrowing the aperture may comprise the step of implanting the prosthetic implant into a patient's pelvis.

While this invention has been described as having preferred designs, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A plug for covering an aperture in a prosthetic implant, the prosthetic implant including an annular ridge projecting into the aperture and narrowing the aperture, said plug comprising:
a first end;
a second end;
a head located at the second end of the plug;
a body extending between the head of the plug and the first end of the plug, said body comprising an exterior surface and a longitudinal axis;
a thread that extends from the exterior surface of the body and wraps helically around the body; and
at least one notch in the first end of the plug that interrupts the thread, the plug having a first position within the aperture in which the annular ridge aligns with the plug along the at least one notch and a second position within the aperture in which the thread engages the annular ridge.

2. The plug of claim 1, further comprising a plurality of notches spaced across the first end of the plug.

3. An implant system comprising:
a prosthetic implant comprising:
a bone-contacting surface;
a receiving surface opposite the bone-contacting surface;
at least one aperture extending a length between the bone-contacting surface and the receiving surface; and
a plug sized to be received within the at least one aperture of the prosthetic implant, said plug comprising:
a first end;
a second end;
a body extending between the second end of the plug and the first end of the plug a length substantially equivalent to the length of the aperture, said body comprising an exterior surface and a longitudinal axis;
a thread that wraps helically around the body; and
at least one notch in the body that interrupts the thread.

4. The implant system of claim 3, wherein the prosthetic implant comprises a prosthetic acetabulum.

5. The implant system of claim 3 or 4, wherein:
the prosthetic implant includes an annular ridge projecting into the aperture to narrow the aperture; and
the thread of the plug is configured to engage said annular ridge.

6. The implant system according to any of claims 3 to 5, wherein the first end of the plug and the second end of the plug are essentially flat, whereby the first end of the plug and the second end of the plug avoid projecting substantially beyond the aperture.

7. The implant system according to any of claims 3 to 6 or the plug of claim 1 or 2, wherein:
a plane extends transversely to the longitudinal axis of the body, said plane intersecting the first end of the plug to form an intersection; and
the at least one notch is located entirely within said plane.

8. The implant system according to any of claims 3 to 6 or the plug of claim 1 or 2, wherein:
a plane extends transversely to the longitudinal axis of the body, said plane intersecting the first end of the plug to form an intersection; and
the at least one notch spans a portion of said intersection.

9. The implant system according to any of claims 3 to 6 or the plug of claim 1 or 2, wherein:
a plane extends transversely to the longitudinal axis of the body, said plane intersecting the first end of the plug to form an intersection; and
the at least one notch is located partially within said plane.

10. The implant system or the plug according to any of claims 7 to 9, wherein the plane extends approximately 30 degrees from the longitudinal axis of the body.

11. The implant system or the plug according to any of claims 7 to 9, wherein the plane extends approximately 15 degrees from the longitudinal axis of the body.

12. The implant system or the plug according to any of claims 7 to 11 , wherein the at least one notch comprises a central portion and two end portions, said central portion positioned within the plane and said two end portions positioned outside of the plane.

13. A method of covering an aperture in a prosthetic implant, the prosthetic implant including an annular ridge projecting into the aperture and narrowing the aperture, said method comprising the steps of:
providing a plug comprising a second end, a first end, a threaded portion, and an interrupted thread portion;
positioning the plug within the aperture of the prosthetic implant to align the interrupted thread portion of the plug with the annular ridge; and
turning the plug to engage the annular ridge with the threaded portion of the plug.

14. The method of claim 13, wherein the interrupted thread portion of the plug comprises a notch in the first end of the plug.

15. The method of claim 13 or claim 14, wherein the step of turning the plug comprises positioning the plug entirely within the aperture of the prosthetic implant, whereby the first end of the plug and the second end of the plug avoid projecting substantially beyond the aperture.
